# EUROPEAN PATENT SPECIFICATION

(11) **EP 3 730 940 B1**
(45) Date of publication and mention of the grant of the patent: **22.01.2025**
(21) Application number: 19764647.4
(22) Date of filing: 21.02.2019
(51) Int. Cl.: G01N 33/543, G01N 21/64, G01N 21/05, G01N 33/531, G01N 33/53

(54) **SPECIMEN DILUENT, METHOD FOR PREPARING SAMPLE, SAMPLE, AND SANDWICH METHOD**
PROBENVERDÜNNUNGSMITTEL, VERFAHREN ZUR VORBEREITUNG EINER PROBE, PROBE UND SANDWICH-VERFAHREN
DILUANT D'ANALYTE, ÉCHANTILLON AINSI QUE PROCÉDÉ DE FABRICATION DE CELUI-CI, ET PROCÉDÉ EN SANDWICH

(30) Priority: 07.03.2018 JP 2018040824
(43) Date of publication of application: 28.10.2020
(73) Proprietor: Otsuka Pharmaceutical Co., Ltd., Tokyo 101-8535 (JP)
(72) Inventor: NINOMIYA, Hidetaka, Tokyo 100-7015 (JP); KANEKO, Tomonori, Tokyo 100-7015 (JP); KOJIMA, Shun, Tokyo 100-7015 (JP); KAYA, Takatoshi, Tokyo 100-7015 (JP)
(74) Representative: Gille Hrabal Partnerschaftsgesellschaft mbB Patentanwälte
(86) International application number: PCT/JP2019/006484
(87) International publication number: WO 2019/171967

(56) References cited:
- EP-A1- 2 913 670
- WO-A1-2015/194350
- CN-C- 1 328 583
- JP-A- 2001 255 325
- JP-A- 2006 112 834
- JP-A- H0 566 223
- VISENTIN JONATHAN ET AL: "Deciphering IgM interference in IgG anti-HLA antibody detection with flow beads assays", HUMAN IMMUNOLOGY, NEW YORK, NY, US, vol. 77, no. 11, 20 February 2016 (2016-02-20), pages 1048 - 1054, XP029815293, ISSN: 0198-8859, DOI: 10.1016/J.HUMIMM.2016.02.008
- ADELMANN, K. ET AL.: "Different conformational forms of serum carnosinase detected by a newly developed sandwich ELISA for the measurements of carnosinase concentrations", AMINO ACIDS, vol. 43, 17 February 2012 (2012-02-17), pages 143 - 151, XP035071218, doi:10.1007/s00726-012-1244-8

## Description

### Technical Field

The present invention relates to a specimen diluent for use in a sandwich method, a method for preparing a sample for use in a sandwich method, a sample for use in a sandwich method, and a sandwich method.

### Background Art

Recent studies have revealed that most proteins in a living body are modified by sugar chains, and sugar chains added to proteins play an important role in various life phenomena such as stability of proteins, bonding to hormones, bonding to toxins, virus infection, mycoplasma infection, bacterial infection, protozoan parasitism, fertilization, developmental differentiation, cancer cell metastasis, and apoptosis. It is known that even proteins with the same amino acid sequence and the same name are modified by various types of sugar chains, and the structures of the sugar chains and roles thereof in a living body vary depending on the state of cells that produce the proteins.

Regarding a relationship between the structure of a sugar chain and a disease, for example, it has been reported that various structures of the sugar chains change as cells become cancerous, and the sugar chain is expected to be one of markers that identify cancer.

In order to specifically detect a glycoprotein using a sugar chain as a marker, a protein called a lectin, which has ability to specifically recognize and is bonded to a specific sugar residue in the sugar chain, has been widely used. However, for example, a lectin has lower bonding strength than an antibody and low specificity of bonding to a sugar chain, disadvantageously. Therefore, a sandwich method has been performed in which specificity is increased by using a lectin and an antibody in combination, and a protein (glycoprotein) having a specific sugar chain as a detection target substance can be quantitatively analyzed.

In the sandwich method, an antibody that is specifically bonded to a protein portion of a glycoprotein is fixed to a substrate and used as an immobilized antibody, and a lectin to be mainly bonded to a sugar residue contained in a sugar chain portion of the glycoprotein is linked to a labeling agent and used as a labeled lectin.

However, in a case of using this method, in a system containing a large amount of contaminants such as a glycoprotein and a glycolipid other than a detection target substance, such as blood or urine used as a specimen in ordinary clinical diagnosis, a labeled lectin is bonded not only to a sugar chain of the detection target substance but also to a sugar chain of the contaminants. Therefore, noise (background) is increased, and performance in sensitivity and quantification is deteriorated. Therefore, improvement has been made for use in general clinical diagnosis using blood or the like as a specimen.

The present applicants have found that noise is increased because several types of glycoproteins such as immunoglobulin M (IgM) contained in blood (serum) used as a specimen are nonspecifically bonded to a measurement area as contaminants, and have reduced the noise by cleaving a disulfide bond between the contaminants and a lectin with a reducing agent. (For example, Patent Literature 1)

### Citation List

### Patent Literature

Patent Literature 1: WO 2015/194350 A
Further prior art references are EP 2 913 670 A, VISENTIN JONATHAN ET AL: "Deciphering IgM interference in IgG anti-HLA antibody detection with flow beads assays", HUMAN IMMUNOLOGY, vol. 77, no. 11, pages 1048 to 1054 and CN 1 328 583 C.

EP 2 913 670 A discloses a diluent for use in a standard sandwich ELISA assay, comprising EDTA and reducing agents comprising a thiol group, such as glutathione, cysteine or penicillamine. VISENTIN JONATHAN ET AL. and CN 1 328 583 C further disclose diluents comprising DTT and EDTA for a sandwich assay.

### Summary of Invention

### Technical Problem

Further intensive studies were made on the basis of Patent Literature 1 and the like, and an object of the present invention is to provide, in order to quantify a detection target substance contained in a specimen, a specimen diluent capable of reducing an influence of contaminants, that is, noise with respect to a quantitative value of a detection target to obtain favorable signal sensitivity, a method for preparing a sample, a sample, and a sandwich method.

### Solution to Problem

That is, the present invention provides a specimen diluent, a method for preparing a sample, a sample, and a sandwich method, for example, described in [1] to [14] below.
[1] A specimen diluent used in a sandwich method, the specimen diluent containing 10 mM to 500 mM of nitrilotriacetic acid or salts thereof and 10 mM to 500 mM of dithiothreitol.
[2] The specimen diluent according to [1], having a pH of 6.0 to 9.0.
[3] The specimen diluent according to [1] or [2], in which the sandwich method is performed by surface plasmon-field enhanced fluorescence spectroscopy (SPFS) or enzyme-linked immuno sorbent assay (ELISA).
[4] The specimen diluent according to any one of [1] to [3], in which the sandwich method uses at least one of an antibody and a lectin.
[5] The specimen diluent according to any one of [1] to [4], in which the sandwich method uses at least one of a fluorescent dye, fluorescent nanoparticles, aggregated nanoparticles, magnetic beads, an enzyme/coenzyme, a chemiluminescent substance, and a radioactive substance.
[6]
   The specimen diluent according to any one of [1] to [5], containing an inorganic reducing agent.
[7] The specimen diluent according to [6], in which the inorganic reducing agent is at least one selected from the group consisting of sodium metabisulfite (SM), sodium sulfite (SS), hydrosulfite, and thiourea dioxide.
[8] A method for preparing a sample used in a sandwich method, the method including a step of mixing 1 to 100 µg of a specimen diluent per µg of a specimen, in which the specimen diluent contains 10 mM to 500 mM of nitrilotriacetic acid or salts thereof and 10 mM to 500 mM of dithiothreitol.
[9] The method for preparing a sample according to [10], in which the specimen is a biologically originated substance, and contains a glycoprotein as a detection target substance contained in the specimen.
[10] A sample used in a sandwich method, the sample containing a specimen and the specimen diluent according to any one of [1] to [7], in which the amount of the specimen diluent per µg of the specimen is 1 to 100 µg.
[11] The sample according to [10], in which the specimen is a biologically originated substance, and contains a glycoprotein as a detection target substance contained in the specimen.
[12] A sandwich method performed by surface plasmon-field enhanced fluorescence spectroscopy (SPFS) or enzyme-linked immuno sorbent assay (ELISA) using the sample according to [10] or [11].

### Advantageous Effects of Invention

The present invention can provide, in order to quantify a detection target substance contained in a specimen, a specimen diluent capable of reducing an influence of contaminants, that is, noise with respect to a quantitative value of a detection target to obtain favorable signal sensitivity, a method for preparing a sample, a sample, and a sandwich method.

### Brief Description of Drawings

Fig. 1 illustrates results of Comparative Example 1 and Reference Example 1 (reference examples are not according to the current invention). The horizontal axis indicates a signal value of a noise component, and the vertical axis indicates a signal value of a signal component.
Fig. 2 illustrates results of Comparative Example 2 and Example 1. The horizontal axis indicates a signal value of a noise component, and the vertical axis indicates a signal value of a signal component.

### Description of Embodiments

Next, the present invention will be described specifically.

### <<Specimen diluent>>

A specimen diluent of the present invention is a specimen diluent used in a sandwich method, the specimen diluent containing 10 mM to 500 mM of nitrilotriacetic acid or salts thereof and 10 mM to 500 mM of dithiothreitol.

The specimen diluent of the present invention contains 10 mM to 500 mM, preferably 30 mM to 100 mM of nitrilotriacetic acid or salts thereof because it is necessary not to inactivate a protein of a signal-related component. In addition, the specimen diluent of the present invention contains 10 mM to 500 mM, preferably 50 mM to 200 mM of dithiothreitol t.

Furthermore, according to one of preferable aspects, the specimen diluent of the present invention contains an inorganic reducing agent as an optional component. In a case where the specimen diluent of the present invention contains an inorganic reducing agent, the specimen diluent contains preferably 10 mM to 800 mM, more preferably 100 mM to 200 mM of the inorganic reducing agent.

For example, when a specimen is a biologically originated substance, and a detection target substance contained in the specimen is quantified by a sandwich method such as surface plasmon-field enhanced fluorescence spectroscopy (SPFS), the specimen may be diluted to obtain a measurement sample. Such a solution used for dilution is referred to as a specimen diluent in the present invention.

The specimen diluent is preferably a buffer solution from a viewpoint of stable pH. Examples of the buffer solution include an acetate buffer solution, a phosphate buffer solution, a Tris buffer solution, a MES buffer solution, a HEPES buffer solution, a citrate buffer solution, a citrate phosphate buffer solution, and a borate buffer solution. As the buffer solution, a phosphate buffered saline (PBS) solution, Tris buffered saline (TBS-T), and a MES buffered saline, which are substantially isotonic with body fluid, are preferable.

The pH of the specimen diluent is preferably 6.0 to 9.0, and more preferably 6.5 to 8.5.

Note that a method for measuring the pH is not particularly limited. It is only required to measure the pH under appropriate measuring conditions by a general pH measuring method (for example, a glass electrode method).

The specimen diluent may contain a surfactant and the like in order to improve dissolution stability of a specimen. Examples of the surfactant include Tween 20 (polyoxyethylene sorbitan monolaurate), Triton X100 (4-(1,1,3,3-tetramethylbutyl) phenyl-polyethylene glycol), and Span 80 (sorbitan monooleate). Tween 20 is a preferable surfactant. In a case where the specimen diluent contains a surfactant, the concentration of the surfactant is preferably 0.00001 to 0.1% by mass with respect to 100% by mass of the specimen diluent.

In a case where a specimen is a biologically originated substance, it is preferable to add 1 to 100 µg of the specimen diluent to 1 µg of the specimen.

The present inventors presumed that a reason why noise with respect to a quantitative value could be reduced to obtain favorable signal sensitivity by using the specimen diluent of the present invention when a detection target substance was quantified might be as follows.

In a case where a compound having a thiol group was used, it was known that noise derived from contaminants such as IgM could be reduced by reductively cleaving a disulfide bond (-S-S-bond) included in the contaminants such as IgM. However, the present inventors found that measurement sensitivity also decreased at the same time.

In a case where a compound having a thiol group was used, the present inventors presumed that measurement sensitivity decreased because not only a disulfide bond of contaminants such as IgM but also a disulfide bond in a capture substance (for example, IgG such as an anti-PSA antibody) for capturing a detection target substance, constituting a solid phase in a sandwich method, was cleaved at the same time. In the present invention, by using nitrilotriacetic acid or salts thereof in addition to dithiothreitol as a compound having a thiol group, the present inventors presumed that cleavage of the disulfide bond in the capture substance could be suppressed by the following mechanism, resulting in favorable signal sensitivity.

The present inventors presumed that nitrilotriacetic acid or salts thereof interacted with dithiothreitol to form a complex, the formation of the complex reduced reactivity of cleavage of a disulfide bond by the thiol, and a disulfide bond was cleaved in contaminants such as IgM having a disulfide bond in a portion closer to a surface, but a disulfide bond was not cleaved in the capture substance. Note that a fact that the specimen diluent of the present invention dramatically reduces odor of dithiothreitol as compared with a case where nitrilotriacetic acid or salts thereof are not used is also one of grounds for presuming that nitrilotriacetic acid or salts thereof interact with dithiothreitol.

From those described above, the present inventors consider that by using the specimen diluent of the present invention in a sandwich method, it is possible to perform quantification with excellent sensitivity without causing signal deterioration.

### < Nitrilotriacetic acid or salts thereof >

The specimen diluent of the present invention contains nitrilotriacetic acid or salts thereof. The present inventors estimate that noise may be reduced without causing signal deterioration because the nitrilotriacetic acid or salts thereof interact with dithiothreitol. In addition, the present inventors estimate that noise may be reduced without causing signal deterioration because in a complex measurement system in which various substances during quantification of a detection target substance (for example, a metal ion, an ionic component, a detection target substance, a capture substance, and contaminants) coexist, the compound having a structure represented by formula (I) below also contributes to enhancement of dissolution stability of these substances.
Examples of the salts of nitrilotriacetic acid include an alkali metal salt and an ammonium salt. An alkali metal salt is preferable, and a sodium salt is more preferable from a viewpoint of dissolution stability.

### < Dithiothreitol >

The specimen diluent of the present invention contains dithiothreitol.

The present inventors presumed that the dithiothreitol compound reduced and cleaved an -S-S- bond in contaminants, particularly in IgM by the following mechanism.

The present inventors presumed that nitrilotriacetic acid or salts thereof and dithiothreitol formed a complex to make a molecule huge, and a migration speed thereby decreased. Then, the present inventors presumed that a reducing action selectively acted on contaminants (particularly IgM) having larger molecules than a capture substance for capturing a detection target substance (for example, IgG such as an anti-PSA antibody), and only the contaminants might be thereby reduced without indiscriminate reduction to reduce noise.

### <Inorganic reducing agent>

The specimen diluent of the present invention may optionally contain an inorganic reducing agent.

The inorganic reducing agent is, for example, at least one selected from the group consisting of sodium metabisulfite (SM), sodium sulfite (SS), hydrosulfite, and thiourea dioxide, and is preferably at least one selected from the group consisting of sodium metabisulfite (SM) and sodium sulfite (SS).

The inorganic reducing agent may be used singly or in combination of two or more types thereof.

The present inventors estimate that noise may be reduced because the inorganic reducing agent reduces and cleaves an -S-S- bond in contaminants, particularly on a surface of a highly hydratable protein.

### <<Sandwich method>>

A sandwich method is one of qualitative and quantitative methods for proteins using an antibody. The sandwich method is a method for immobilizing, in advance, a complementary substance (antibody, for example, IgG such as an anti-PSA antibody) for a detection target substance (antigen) on a measurement area of a well plate, magnetic particles, a sensor chip, or the like, capturing the detection target substance by an immune reaction, and subsequently bonding a labeled substance to be specifically bonded to the detection target substance thereto for detection.

The antibody is not particularly limited as long as being able to capture the detection target substance. The detection target substance will be described in detail later. For example, in a case where a prostate-specific antigen (PSA) is used as a detection target substance, an anti-PSA antibody can be used.

The labeled substance is not particularly limited as long as being specifically bonded to the detection target substance. For example, in a case where the detection target substance has a sugar chain, a lectin-labeled substance can be used.

There are various types of lectins, but it is only required to use a lectin suitable for a purpose. For labeling, a fluorescent dye, fluorescent nanoparticles, aggregated nanoparticles, magnetic beads, an enzyme/coenzyme, a chemiluminescent substance, a radioactive substance, and the like can be used. For example, in a case where the prostate-specific antigen (PSA) is used as the detection target substance, it is only required to label a lectin such as wisteria floribunda lectin (WFA), soybean lectin (SBA), or Trichosanthes japonica lectin (TJA-II) for use.

The present invention relates to a specimen diluent that can be used in such a sandwich method.

The sandwich method of the present invention is performed, for example, by surface plasmon-field enhanced fluorescence spectroscopy (SPFS) or enzyme-linked immuno sorbent assay (ELISA).

In quantification by the sandwich method of the present invention, by creating a calibration curve using signal intensity obtained from a biologically originated substance whose concentration is known as a specimen and applying signal intensity obtained from a detection target substance thereto, a concentration can be determined.

The sandwich method of the present invention can specifically detect an extremely small amount of a detection target substance, and therefore is preferably applied when a highly sensitive fluorescence measurement such as surface plasmon-field enhanced fluorescence spectroscopy (SPFS) is performed.

For example, in a sandwich method in which quantification is performed using SPFS, preferably, an antibody for a detection target substance (antigen) is first immobilized in advance on a measurement area of a sensor chip or the like, and the detection target substance is captured by an immune reaction. Then, a sandwich method is preferable in which a substance obtained by linking a labeling agent to a substance that is specifically bonded to the detection target substance is detected and quantified.

### <Surface plasmon-field enhanced fluorescence spectroscopy>

Surface plasmon-field enhanced fluorescence spectroscopy (SPFS) obtains an effect of enhancing an electric field of surface plasmon light by generating surface plasmon light (compressional wave) on a surface of a metal thin film under a condition that excitation light such as laser light emitted from a light source undergoes attenuated total reflectance (ATR) on the surface of the metal thin film to increase the amount of photons possessed by the excitation light emitted from the light source by dozens of times to several hundreds of times.

A ligand molecule such as an antibody is fixed near the surface of the metal thin film, an antigen is captured there by an immune reaction, and then a fluorescence-labeling molecule is bonded thereto. Then, due to the above effect of enhancing an electric field, a fluorescent substance labeled with fluorescence is efficiently excited, and by observing this fluorescence, it is possible to detect an extremely small amount of antigen with an extremely low concentration.

A preferable example of an embodiment of the present invention is a case where a specimen is a biologically originated substance and contains a glycoprotein as a detection target substance contained in the specimen. The specimen diluent of the present invention is mixed with the specimen to obtain a measurement solution, and quantification can be performed by surface plasmon-field enhanced fluorescence spectroscopy (SPFS).

For example, in order to quantify a glycoprotein as an extremely small amount of an antigen contained in such a measurement sample as described above using surface plasmon excitation-enhanced fluorescence spectroscopy (SPFS), it is only required to appropriately select an antibody that is specifically bonded to a protein portion of the glycoprotein as an immobilized antibody on a measurement area of a surface of a metal thin film to be used for capturing without any particularly limitation. It is preferable to link a labeling agent such as fluorescence to a substance whose bonding target is a sugar residue contained in a sugar chain portion of the captured glycoprotein to be used for detection, and a labeling method is not particularly limited. For example, a lectin labeled with a fluorescent dye (referred to as a fluorescence-labeled lectin) can be used. Manufacturing is performed using a commercially available fluorescent substance labeling kit "Alexa Fluor (trademark) 647 protein labeling kit" (manufactured by Invitrogen) to obtain a fluorescence-labeled lectin.

### <Enzyme immunoassay>

Typical examples of an enzyme that can be used in enzyme-linked immuno sorbent assay (ELISA) are horseradish-derived peroxidase (HRP) and alkaline phosphatase (ALP). It is only required to use HRP and ALP in combination with appropriate substrates corresponding thereto, respectively.

### <<Specimen>>

In the present invention, a specimen is preferably a biologically originated substance, and it is preferable to use a specimen that may contain a detection target substance and contaminants. The specimen that may contain a detection target substance and contaminants may be a specimen actually containing a detection target substance and contaminants or a specimen not containing a detection target substance and contaminants actually. Specifically, for example, the specimen may be a specimen that is highly likely to contain a detection target substance (and contaminants) and is derived from a patient with a specific disease, or may be a specimen that is less likely to contain a detection target substance (and contaminants) and is derived from a healthy person. A target from which a specimen is collected is typically a human, but may be a non-human mammal such as a mouse, a rat, a guinea pig, a rabbit, a goat, a cat, a dog, a pig, or a monkey, which is a model animal for a human disease.

Examples of the specimen include blood, urine, spinal fluid, saliva, cells, tissues, organs, and preparations thereof (for example, biopsy specimen). For example, blood or urine is preferable because of being a specimen containing a glycoprotein that can be used as a diagnostic marker.

A liquid specimen such as blood, serum, plasma, urine, spinal fluid or saliva can be used by being diluted with an appropriate buffer solution. A solid specimen such as cells, tissues, or organs is homogenized with an appropriate buffer solution of about 2 to 10 times the volume of the solid specimen, and the resulting suspension or a supernatant thereof can be used as it is or by being further diluted.

A specimen such as blood or serum is diluted with the specimen diluent of the present invention and can be used as a measurement sample in a sandwich method.

As a preferable example of the embodiment of the present invention, blood is used as a specimen. Here, the blood may be whole blood, or serum or plasma prepared from the whole blood. An anticoagulant, a diluent (buffer solution and the like), a reagent, and the like may be added to the blood.

### <Detection target substance>

A detection target substance contained in a specimen is preferably a glycoprotein. The glycoprotein as the detection target substance is, for example, a marker molecule contained in a specimen and used for pathological diagnosis. For example, a cancer antigen/tumor marker such as a prostate-specific antigen (PSA), α-fetoprotein (AFP), or a carcinoembryonic antigen (CEA) is suitable, and another glycoprotein such as a signal transduction substance or a hormone can also be used as the detection target substance. For example, prostate-specific antigen (PSA) can be obtained from a human prostate cancer cell culture line (LNCaP).

The detection target substance is preferably a glycoprotein containing a bonding site to a substance labeled with a fluorescent dye or the like and a bonding site to a capture substance (for example, an antibody) in the same domain, that is, a glycoprotein in which a domain containing a bonding site to a substance labeled with a fluorescent dye or the like is not separated from a domain containing a bonding site to a capture substance by an action of components in the specimen diluent.

### <<Method for preparing sample>>

A method for preparing a sample of the present invention is a method for preparing a sample used in a sandwich method, the method including a step of mixing 1 to 100 µg of a specimen diluent per µg of a specimen, in which the specimen diluent contains 10 mM to 500 mM of a compound having a structure represented by the following formula (I) and 10 mM to 500 mM of a compound having a thiol group.

The nitrilotriacetic acid or salts thereof and dithiothreitol are as described above.

This manufacturing method may include a step of adding a buffer, a surfactant, and the like in addition to the specimen and the specimen diluent.

Note the sample is preferably manufactured at room temperature (15 to 25°C). It is preferable to subject the sample to measurement one second to 30 minutes after manufacturing because noises derived from contaminants can be reduced.

### «Sample»

A sample used in the present invention is a sample used in a sandwich method, the sample containing a specimen and a specimen diluent, in which the amount of the specimen diluent per µg of the specimen is 1 to 100 µg.

The sample of the present invention is prepared using the above-described specimen diluent of the present invention, and therefore can reduce an influence derived from contaminants, that is, noise with respect to a quantitative value of a detection target to obtain favorable signal sensitivity. Thus, the sample of the present invention is preferable.

### Examples

### <<Experiment 1>>

### [Comparative Example 1 and Reference Example 1]

### (Preparation of specimen diluent)

A phosphate-buffered saline (PBS) solution containing DTT, EDTA, or both EDTA and DTT was prepared so as to have each of the concentrations illustrated in Table 1 to obtain a specimen diluent. Note that a specimen diluent containing only PBS was used as a control.

Table 1 illustrates the type and concentration of a specimen diluent for each case in Comparative Example and Reference Example.

### (Preparation of measurement sample)

To 100 µL of the above specimen diluent, 20 µL of PSA free pool serum (normal human pool serum, Kohjin Bio Co., Ltd., confirmed by ELISA to have a PSA concentration of 1 pg/mL (0.001 ng/mL) or less) was added and mixed, and used as a signal measurement sample of a noise component.

To the PSA free pool serum, LNCaP (human prostate cancer cell culture line, DS Pharma Biomedical) culture supernatant was added such that the total PSA concentration was 2 ng/mL to prepare PSA-containing serum. Note that the PSA concentration was adjusted using a PSA measurement kit (Total PSA/Abbott, Abbott Japan Co., Ltd.).

To 100 µL of the above specimen diluent, 20 µL of the above PSA-containing serum was added and mixed, and used as a signal measurement sample of a signal component.

### (Preparation of sensor chip for SPFS)

In order to form a membrane (CMD membrane) made of carboxymethyl dextran on a sensor chip for SPFS, 0.8 mL of 25 mM MES buffered saline and 0.8 mL of 10 mM NaCl solution (pH 6.0) were dropped on the sensor chip for SPFS, and were allowed to react for 20 minutes.

The MES buffered saline was prepared by mixing 0.5 mM of N-hydroxysuccinimide (NHS), 0.5 mM of water-soluble carbodiimide (WSC), and 1 mg/mL of carboxymethyl dextran "CMD-500-06I4" (Meito Sangyo Co., Ltd.: average molecular weight 500,000, substitution degree: 0.51).

Into a channel on the sensor chip, 5 mL of a PBS solution containing 50 mM of N-hydroxysuccinimide (NHS) and 100 mM of water-soluble carbodiimide (WSC) was introduced, and circulated for 20 minutes at a flow rate of 500 µL/min to convert a carboxyl group of CMD into an active ester. Thereafter, 2.5 mL of an anti-PSA antibody solution was circulated for 30 minutes at a flow rate of 500 µL/min to bond the antibody to the active ester group of CMD, thereby immobilizing the anti-PSA antibody on the sensor chip to establish a measurement area.

The above measurement sample was injected into the sensor chip on which the anti-PSA antibody was immobilized and was allowed to react with the measurement area while the solution was reciprocated for 30 minutes.

Thereafter, a Tris-buffered saline (TBS-T) solution containing 0.05 wt% of Tween (registered trademark) 20 was introduced into the channel, and the solution was reciprocated by pipetting for three minutes to wash the measurement area.

### (Preparation of fluorescence-labeled lectin)

A fluorescence-labeled lectin (Alexa Fluor 647-labeled WFA) was prepared using a fluorescent substance labeling kit "Alexa Fluor (trademark) 647 protein labeling kit" (manufactured by Invitrogen). 100 µg of wisteria floribunda lectin (WFA) "L-1350" (Vector Laboratories, Inc.), 0.1 M sodium bicarbonate, and Alexa Fluor 647 reactive dye were mixed and allowed to react at room temperature for one hour. Thereafter, gel filtration chromatography and ultrafiltration were performed to remove Alexa Fluor 647 reactive dye that had not been used for labeling, and the fluorescence-labeled WFA was recovered. The absorbance of the obtained fluorescence-labeled WFA solution was measured, and the concentration thereof was quantified. The fluorescence-labeled WFA solution was diluted with PBS to adjust the concentration thereof to 1 µg/mL.

### (Measurement of signal value)

Into the sensor chip, 100 µL of the 1 µg/mL fluorescence-labeled lectin (Alexa Fluor 647-labeled WFA) solution prepared above was introduced. The introduced solution was reciprocated by pipetting for 10 minutes to be allowed to react with the measurement area.

Thereafter, a TBS-T solution was introduced into the measurement area, and the solution was reciprocated for three minutes. Thereafter, the channel and the inside of the sensor chip were washed. Then, irradiation with excitation light was performed in a state where the channel was filled with the TBS-T solution, and the fluorescence emission intensity of Alexa Fluor 647 was measured. The measured value was used as a signal value.

### (Calculation of S/N ratio)

An S/N ratio was calculated from the obtained signal value using the following formula (II). [Signal value of signal component (PSA concentration 2 ng/mL)]/[Signal value of noise component (PSA concentration 0 ng/mL)]

Table 1 illustrates a signal value and an S/N ratio for each case of Comparative Example 1 and Reference Example 1.

### [Table 1]

**Table 1**

| | | Type and concentration of specimen diluent | Signal component PSA 2 ng/mL serum | Noise component PSA 0 ng/mL serum | S/N ratio |
|---|---|---|---|---|---|
| Comparative Example 1 | Reference | PBS (control) | 670604 | 233037 | 2.9 |
| | 1 | DTT 50 mM | 310568 | 33501 | 9.3 |
| | 2 | DTT 100 mM | 283799 | 17985 | 15.8 |
| | 3 | DTT 200 mM | 187601 | 15033 | 12.5 |
| | 4 | EDTA 50 mM | 441600 | 75694 | 5.8 |
| | 5 | EDTA 100 mM | 380183 | 38363 | 9.9 |
| | 6 | EDTA 150 mM | 343672 | 36313 | 9.5 |
| Reference Example 1* | 1 | DTT 50 mM + EDTA 50 mM | 341791 | 11783 | 29.0 |
| | 2 | DTT 100 mM + EDTA 50 mM | 326250 | 8594 | 38.0 |
| | 3 | DTT 50 mM + EDTA 100 mM | 312627 | 9858 | 31.7 |
| | 4 | DTT 100 mM + EDTA 100 mM | 300925 | 9882 | 30.5 |

| | | | | | |
|---|---|---|---|---|---|
| A graph of the signal values in Table 1 is illustrated in Fig. 1. *Reference examples are not according to the current invention | | | | | |

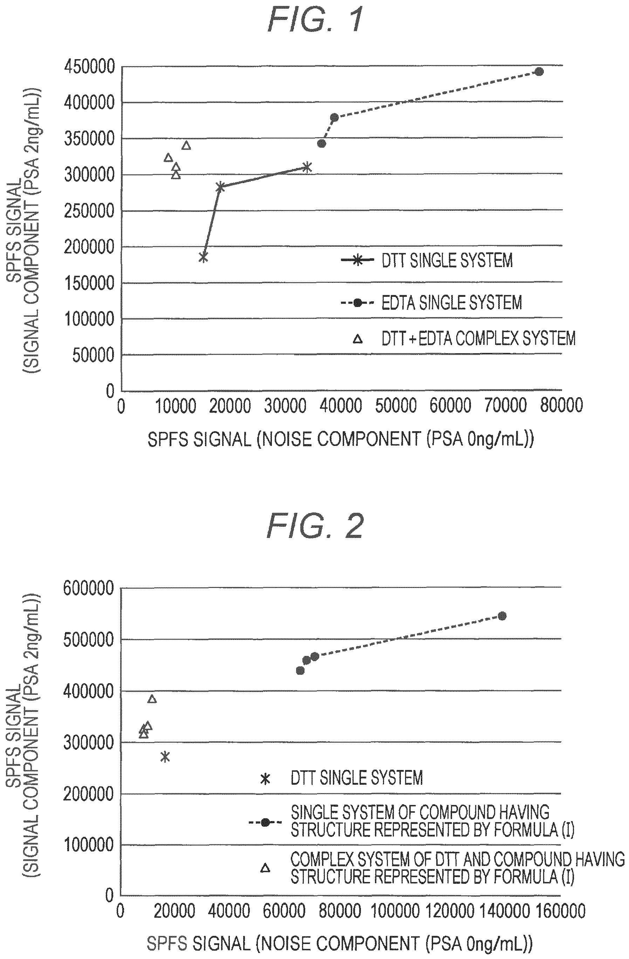

In Fig. 1, the horizontal axis indicates a signal value of a noise component, and the vertical axis indicates a signal value of a signal component. For each of cases 1 to 4 of Reference Example 1, a triangle is indicated as a DTT + EDTA complex system in the figure. For each of cases 1 to 3 of Comparative Example 1, an asterisk is indicated as a DTT single system in the figure. For each of cases 4 to 6 of Comparative Example 1, a black circle is indicated as an EDTA single system in the figure.

The results in Table 1 and Fig. 1 will be examined. In a case where DTT had a concentration of 100 mM or more, a signal value of a noise component did not decrease, and a signal value of a signal component significantly decreased, resulting in poor sensitivity. In a case where EDTA had a concentration of 100 mM or more, an effect of decreasing a signal value of a noise component was not improved, and a signal value of a signal component also decreased, resulting in poor sensitivity. Meanwhile, when both DTT and EDTA were used, surprisingly, an effect of sufficiently decreasing a noise value of a noise component was observed while a signal value of a signal component was excellent.

The above results indicate that the case of using both a compound having a structure represented by formula (I) and a compound having a thiol group has a lower signal value of a noise component, reduces noise due to a surprising combined effect of the compound having a structure represented by formula (I) and the compound having a thiol group, and has favorable signal sensitivity as compared with the case of singly using the compound having a structure represented by formula (I) or the compound having a thiol group.

### <<Experiment 2»

### [Comparative Example 2 and Example 1]

### (Preparation of specimen diluent)

A PBS solution containing DTT, EDTA, GEDTA, NTA, or DTPA was prepared so as to have each of the concentrations illustrated in Table 2 and used as a specimen diluent of Comparative Example 2.

A PBS solution containing DTT and any one of EDTA, GEDTA, NTA, and DTPA in combination was prepared so as to have each of the concentrations illustrated in Table 2 and used as a specimen diluent of Example 1. Note that a specimen diluent containing only PBS was used as a control.

### [Table 2]

**Table 2**

| | | Type and concentration of specimen diluent | Signal component PSA 2 ng/mL serum | Noise component PSA 0 ng/mL serum | S/N ratio |
|---|---|---|---|---|---|
| Comparative Example 2 | Reference | PBS (control) | 667740 | 237083 | 2.8 |
| | 1 | DTT 100 mM | 276056 | 16819 | 16.4 |
| | 2 | EDTA 50 mM | 444362 | 66787 | 6.7 |
| | 3 | GEDTA 50 mM | 469135 | 71924 | 6.5 |
| | 4 | NTA 50 mM | 544507 | 142061 | 3.8 |
| | 5 | DTPA 50 mM | 462576 | 69249 | 6.7 |
| Example 1 | 1* | DTT 100 mM + EDTA 50 mM | 321855 | 8506 | 37.8 |
| | 2* | DTT 100 mM + GEDTA 50 mM | 337546 | 9936 | 34.0 |
| | 3 | DTT 100 mM + NTA 50 mM | 330139 | 8343 | 39.6 |
| | 4* | DTT 100 mM + DTPA 50 mM | 389922 | 11748 | 33.2 |

| | | | | | |
|---|---|---|---|---|---|
| *: reference examples, not according to the invention | | | | | |

A signal value and an S/N ratio were obtained in a similar manner to Experiment 1 except for the above preparation of the specimen diluent.

Results thereof are illustrated in Table 2.

A graph of the signal values in Table 2 is illustrated in Fig. 2.

In Fig. 2, the horizontal axis indicates a signal value of a noise component, and the vertical axis indicates a signal value of a signal component. For each of cases 1 to 4 of Example 1, a triangle is indicated as a complex system including DTT and a compound having a structure represented by formula (I) in the figure. For case 1 of Comparative Example 2, an asterisk is indicated as a DTT single system in the figure. For each of cases 2 to 5 of Comparative Example 2, a black circle is indicated as a single system of a compound having a structure represented by formula (I) in the figure.

### <<Experiment 3»

### [Comparative Example 3 and Reference Example 2]

### (Preparation of specimen diluent)

A PBS solution containing DTT, L-cysteine, thioglycolic acid, or mercaptoethanol was prepared so as to have each of the concentrations illustrated in Table 3 and used as a specimen diluent of Comparative Example 3.

A PBS solution containing EDTA and any one of DTT, L-cysteine, thioglycolic acid, and mercaptoethanol in combination was prepared so as to have each of the concentrations illustrated in Table 3 and used as a specimen diluent of Reference Example 2. Note that a specimen diluent containing only PBS was used as a control.

### [Table 3]

**Table 3**

| | | Type and concentration of specimen diluent | Signal component PSA 2 ng/mL serum | Noise component PSA 0 ng/mL serum | S/N ratio | S/N improvement effect % Complex formulation/single formulation |
|---|---|---|---|---|---|---|
| Comparative Example 3 | Reference | PBS (control) | 667740 | 237083 | 2.8 | - |
| | 1 | DTT 100 mM | 276055 | 16819 | 16.4 | - |
| | 2 | L-cysteine 100 mM | 357974 | 79924 | 4.5 | - |
| | 3 | Thioglycolic acid 100 mM | 261110 | 27409 | 9.5 | - |
| | 4 | Mercaptoethanol 100 mM | 404323 | 67015 | 6.0 | - |
| Reference Example 2* | 1 | DTT 100 mM + EDTA 50 mM | 322649 | 8454 | 38.2 | 233% |
| | 2 | L-cysteine 100 mM + EDTA 50 mM | 340372 | 29084 | 11.7 | 261% |
| | 3 | L-cysteine 200 mM + EDTA 50 mM | 285279 | 23506 | 12.1 | 271% |
| | 4 | Thioglycolic acid 100 mM + EDTA 50 mM | 312784 | 20479 | 15.3 | 160% |
| | 5 | Thioglycolic acid 200 mM + EDTA 50 mM | 298523 | 14563 | 20.5 | 215% |
| | 6 | Mercaptoethanol 100 mM + EDTA 50 mM | 380987 | 25794 | 14.8 | 245% |
| | 7 | Mercaptoethanol 200 mM + EDTA 50 mM | 307856 | 17652 | 17.4 | 289% |

| | | | | | | |
|---|---|---|---|---|---|---|
| *Reference examples are not according to the current invention | | | | | | |

A signal value and an S/N ratio were obtained in a similar manner to Experiment 1 except for the above preparation of the specimen diluent.

An S/N improvement effect % was calculated from the obtained S/N ratio using the following formula (III). S/N improvement effect % = (compound having thiol group + S/N ratio of EDTA)/(S/N ratio of compound having thiol group) × 100

Results thereof are illustrated in Table 3.

### <<Experiment 4»

### [Comparative Example 4 and Reference Example 3]

### (Preparation of specimen diluent)

A PBS solution containing sodium metabisulfite (SM) or sodium sulfite (SS) was prepared so as to have each of the concentrations illustrated in Table 4 to obtain a specimen diluent of Comparative Example 4.

A PBS solution containing DTT and EDTA and either sodium metabisulfite (SM) or sodium sulfite (SS) in combination was prepared so as to have each of the concentrations illustrated in Table 4 to obtain a specimen diluent of Reference Example 3. Note that a specimen diluent containing only PBS was used as a control.

### [Table 4]

**Table 4**

| | | Type and concentration of specimen diluent | Signal component PSA 2 ng/mL serum | Noise component PSA 0 ng/mL serum | S/N ratio | S/N improvement effect % Complex formulation/single formulation |
|---|---|---|---|---|---|---|
| Comparative Example 4 | Reference | PBS (control) | 667740 | 287083 | 2.8 | - |
| | 1 | SM 200 mM | 217534 | 12187 | 17.9 | - |
| | 2 | SS 200 mM | 199839 | 13150 | 15.2 | - |
| Reference Example 3** | 1 | DTT 25 mM + EDTA 50 mM + SM 200 mM | 133519 | 3842 | 34.8 | 195% |
| | 2 | DTT 50 mM + EDTA 50 mM + SM 200 mM | 121981 | 3341 | 36.5 | 205% |
| | 3 | DTT 25 mM + EDTA 50 mM + SS 200 mM | 140579 | 4939 | 28.5 | 187% |
| | 4 | DTT 25 mM + EDTA 50 mM + SS 200 mM | 130584 | 4124 | 31.7 | 208% |

| | | | | | | |
|---|---|---|---|---|---|---|
| *SM: sodium metabisulfite, SS: sodium sulfite ** Reference examples are not according to the current invention | | | | | | |

A signal value, an S/N ratio, and an S/N improvement effect % were obtained in a similar manner to Experiment 3 except for the above preparation of the specimen diluent. Results thereof are illustrated in Table 4.

The results in Table 4 will be examined. In a case where DTT and EDTA and either SM or SS are contained in combination, an effect of decreasing a signal value of a noise component is remarkably excellent, and therefore this suggests that favorable sensitivity is obtained.

## Claims

1. A specimen diluent for use in a sandwich method, the specimen diluent comprising 10 mM to 500 mM of nitrilotriacetic acid or salts thereof and 10 mM to 500 mM of dithiothreitol.

2. The specimen diluent according to claim 1, having a pH of 6.0 to 9.0.

3. The specimen diluent according to claim 1 or 2, wherein the sandwich method is performed by surface plasmon-field enhanced fluorescence spectroscopy (SPFS) or enzyme-linked immuno sorbent assay (ELISA).

4. The specimen diluent according to any one of claims 1 to 3, wherein the sandwich method uses at least one of an antibody and a lectin.

5. The specimen diluent according to any one of claims 1 to 4, wherein the sandwich method uses at least one of a fluorescent dye, fluorescent nanoparticles, aggregated nanoparticles, magnetic beads, an enzyme/coenzyme, a chemiluminescent substance, and a radioactive substance.

6. The specimen diluent according to any one of claims 1 to 5, comprising an inorganic reducing agent.

7. The specimen diluent according to claim 6, wherein the inorganic reducing agent is at least one selected from the group consisting of sodium metabisulfite (SM), sodium sulfite (SS), hydrosulfite, and thiourea dioxide.

8. A method for preparing a sample for use in a sandwich method, the method comprising mixing 1 to 100 µg of a specimen diluent per µg of a specimen, wherein
the specimen diluent contains 10 mM to 500 mM of nitrilotriacetic acid or salts thereof and 10 mM to 500 mM of dithiothreitol.

9. The method for preparing a sample according to claim 8, wherein the specimen is a biologically originated substance, and contains a glycoprotein as a detection target substance contained in the specimen.

10. A sample for use in a sandwich method, the sample comprising a specimen and the specimen diluent according to any one of claims 1 to 7, wherein
an amount of the specimen diluent per µg of the specimen is 1 to 100 µg.

11. The sample according to claim 10, wherein the specimen is a biologically originated substance, and contains a glycoprotein as a detection target substance contained in the specimen.

12. A sandwich method performed by surface plasmon-field enhanced fluorescence spectroscopy (SPFS) or enzyme-linked immuno sorbent assay (ELISA) using the sample according to claim 10 or 11.

## Patentansprüche

1. Probenverdünnungsmittel zur Verwendung in einem Sandwich-Verfahren, wobei das Probenverdünnungsmittel 10 mM bis 500 mM Nitrilotriessigsäure oder Salze davon und 10 mM bis 500 mM Dithiothreitol umfasst.

2. Das Probenverdünnungsmittel nach Anspruch 1 mit einem pH-Wert von 6,0 bis 9,0.

3. Probenverdünnungsmittel nach Anspruch 1 oder 2, wobei das Sandwich-Verfahren durch oberflächenplasmonenfeldverstärkte Fluoreszenzspektroskopie (SPFS) oder Enzym-linked Immunosorbent Assay (ELISA) durchgeführt wird.

4. Das Probenverdünnungsmittel nach einem der Ansprüche 1 bis 3, wobei bei der Sandwich-Methode mindestens ein Antikörper oder ein Lektin verwendet wird.

5. Das Probenverdünnungsmittel nach einem der Ansprüche 1 bis 4, wobei bei der Sandwich-Methode mindestens einer der folgenden Stoffe verwendet wird: ein fluoreszierender Farbstoff, ein fluoreszierender Nanopartikel, aggregierte Nanopartikel, magnetische Kügelchen, ein Enzym/Koenzym, eine chemilumineszente Substanz und eine radioaktive Substanz.

6. Das Probenverdünnungsmittel nach einem der Ansprüche 1 bis 5, das ein anorganisches Reduktionsmittel enthält.

7. Probenverdünnungsmittel nach Anspruch 6, wobei das anorganische Reduktionsmittel mindestens eines ist, ausgewählt aus der Gruppe bestehend aus Natriummetabisulfit (SM), Natriumsulfit (SS), Hydrosulfit und Thioharnstoffdioxid.

8. Verfahren zur Vorbereitung einer Probe zur Verwendung in einem Sandwich-Verfahren, wobei das Verfahren das Mischen von 1 bis 100 µg eines Probenverdünnungsmittels pro µg einer Probe umfasst, wobei
das Probenverdünnungsmittel 10 mM bis 500 mM Nitrilotriessigsäure oder deren Salze und 10 mM bis 500 mM Dithiothreitol enthält.

9. Verfahren zur Herstellung einer Probe nach Anspruch 8, wobei die Probe eine biologisch entstandene Substanz ist und ein Glykoprotein als in der Probe enthaltene Nachweiszielsubstanz enthält.

10. Probe zur Verwendung in einem Sandwich-Verfahren, wobei die Probe eine Probe und das Probenverdünnungsmittel nach einem der Ansprüche 1 bis 7 umfasst, wobei eine Menge des Probenverdünnungsmittels pro µg der Probe 1 bis 100 µg beträgt.

11. Probe nach Anspruch 10, wobei die Probe eine Substanz biologischen Ursprungs ist und ein Glykoprotein als in der Probe enthaltene Nachweiszielsubstanz enthält.

12. Sandwich-Verfahren, das durch Oberflächenplasmonenfeld-verstärkte Fluoreszenzspektroskopie (SPFS) oder Enzyme-linked Immunosorbent Assay (ELISA) unter Verwendung der Probe nach Anspruch 10 oder 11 durchgeführt wird.

## Revendications

1. Diluant de spécimen à utiliser dans une méthode sandwich, le diluant de spécimen comprenant 10 mM à 500 mM d'acide nitrilotriacétique ou des sels de celui-ci et 10 mM à 500 mM de dithiothréitol.

2. Le diluant de spécimen selon la revendication 1, ayant un pH de 6,0 à 9,0.

3. Le diluant de spécimen selon la revendication 1 ou 2, dans lequel la méthode sandwich est réalisée par spectroscopie de fluorescence améliorée par champ plasmonique de surface (SPFS) ou par essai de sorption immuno enzymatiquement lié (ELISA).

4. Le diluant de spécimen selon l'une quelconque des revendications 1 à 3, dans lequel la méthode sandwich utilise au moins un d'un anticorps et d'une lectine.

5. Le diluant de spécimen selon l'une quelconque des revendications 1 à 4, dans lequel la méthode sandwich utilise au moins un d'un colorant fluorescent, des nanoparticules fluorescentes, des nanoparticules agrégées, des billes magnétiques, d'une enzyme/coenzyme, d'une substance chimiluminescente et d'une substance radioactive.

6. Le diluant de spécimen selon l'une quelconque des revendications 1 à 5, comprenant un agent réducteur inorganique.

7. Le diluant de spécimen selon la revendication 6, dans lequel l'agent réducteur inorganique est au moins un agent choisi dans le groupe constitué du métabisulfite de sodium (SM), du sulfite de sodium (SS), de l'hydrosulfite et du dioxyde de thiourée.

8. Méthode de préparation d'un échantillon à être utilisé dans une méthode sandwich, la méthode comprend mélanger de 1 à 100 µg d'un diluant de spécimen par µg de spécimen, dans laquelle le diluant de spécimen contient de 10 mM à 500 mM d'acide nitrilotriacétique ou des sels de celui-ci et de 10 mM à 500 mM de dithiothréitol.

9. La méthode de préparation d'un échantillon selon la revendication 8, dans laquelle le spécimen est une substance d'origine biologique et contient une glycoprotéine en tant que substance cible de détection contenue dans le spécimen.

10. Échantillon à utiliser dans une méthode sandwich, l'échantillon comprenant un spécimen et le diluant de spécimen selon l'une quelconque des revendications 1 à 7, dans lequel une quantité de diluant de spécimen par µg du spécimen est de 1 à 100 µg.

11. L'échantillon selon la revendication 10, dans lequel l'échantillon est une substance d'origine biologique et contient une glycoprotéine comme substance cible de détection contenue dans le spécimen.

12. Méthode sandwich réalisée par spectroscopie de fluorescence améliorée par champ plasmonique de surface (SPFS) ou par essai de sorption immuno enzymatiquement lié (ELISA) utilisant l'échantillon selon la revendication 10 ou 11.
